(19) Europäisches Patentamt
European Patent Office
Office européen des brevets

(11) EP 1 465 607 B1

(12) FASCICULE DE BREVET EUROPEEN

(45) Date de publication et mention de la délivrance du brevet:
**21.03.2007 Bulletin 2007/12**

(21) Numéro de dépôt: **03701497.4**

(22) Date de dépôt: **08.01.2003**

(51) Int Cl.:
***A61K 9/24*** *(2006.01)* ***A61K 31/495*** *(2006.01)*

(86) Numéro de dépôt international:
**PCT/EP2003/000093**

(87) Numéro de publication internationale:
**WO 2003/057198 (17.07.2003 Gazette 2003/29)**

(54) **COMPOSITIONS PHARMACEUTIQUES A LIBERATION MODIFIEE**

PHARMAZEUTISCHE ZUSAMMENSETZUNG MIT MODIFIZIERTER FREISETZUNG

PHARMACEUTICAL FORMULATIONS WITH MODIFIED RELEASE

(84) Etats contractants désignés:
**AT BE BG CH CY CZ DE DK EE ES FI FR GB GR HU IE IT LI LU MC NL PT SE SI SK TR**

(30) Priorité: **09.01.2002 EP 02000521**

(43) Date de publication de la demande:
**13.10.2004 Bulletin 2004/42**

(73) Titulaire: **UCB FARCHIM S.A.**
**CH-1630 Bulle (CH)**

(72) Inventeurs:
- **BERWAER, Monique**
  **B-6120 Ham-sur-Heure-Nalinnes (BE)**
- **DELEERS, Michel**
  **B-1630 Linkebeek (BE)**
- **FANARA, Domenico**
  **B-4520 Wanze (BE)**
- **GUICHAUX, Anthony**
  **B-1950 Kraainem (BE)**

(74) Mandataire: **Lechien, Monique et al**
**UCB, S.A.,**
**Intellectual Property Department**
**Allée de la Recherche 60**
**1070 Brussel (BE)**

(56) Documents cités:
**EP-A- 1 118 321** **WO-A-98/41194**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

EP 1 465 607 B1

## Description

La présente invention se rapporte à une composition pharmaceutique à libération modifiée permettant d'administrer une quantité efficace d'acide 2-[2-[4-[bis(4-fluorophényl)méthyl]-1-pipérazinyl]éthoxy]acétique et de ses sels pharmaceutiquement acceptables afin d'obtenir à la fois, rapidement une concentration plasmatique efficace ainsi que le maintien d'une concentration minimale efficace sur une période prolongée. La composition à libération modifiée comprend au moins deux fractions contenant le principe actif. La première fraction permet une libération immédiate du principe actif et la seconde permet une libération prolongée du principe actif ainsi que le maintien d'une concentration plasmatique efficace sur une période prolongée. Les compositions obtenues sont particulièrement adaptées pour être administrées en une seule prise journalière.

L'acide 2-[2-[4-[bis(4-fluorophényl)méthyl]-1-pipérazinyl]éthoxy]acétique, également connu et appelé ici du nom d'éflétirizine (DCI : dénomination commune internationale), est le composé de formule suivante:

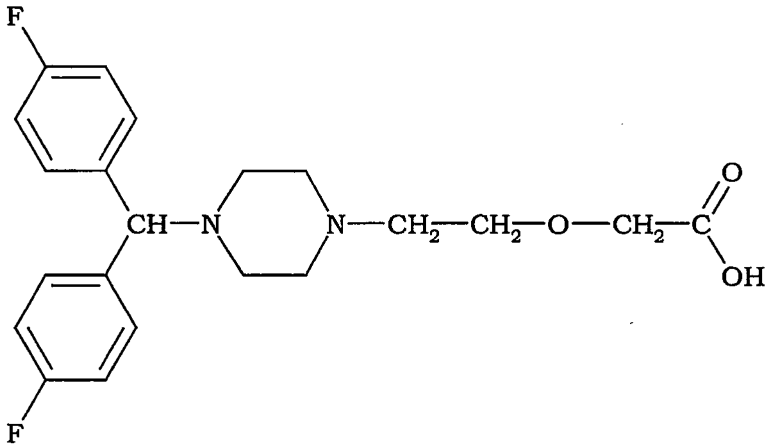

L'éflétirizine est englobée dans la formule générale 1 du brevet européen EP-B-0 058 146 au nom de la demanderesse, qui a trait à des dérivés de benzhydrylpipérazine substitués.

Comme l'acide 2-[2-[4-[(4-chlorophényl)phénylméthyl]-1-pipérazinyl]éthoxy]acétique, également connu et appelé ici du nom de cétirizine, on a constaté que l'éflétirizine possédait d'excellentes propriétés antihistaminiques. Elle appartient à la catégorie pharmacologique des inhibiteurs des récepteurs $H_1$ de l'histamine de seconde génération et présente une grande affinité et une grande sélectivité in vitro pour les récepteurs $H_1$. Comme la cétirizine, elle est utile comme antiallergique, antihistaminique, bronchodilatateur et agent antispasmodique. Des études cliniques récentes ont montré l'utilité de l'éflétirizine lorsqu'elle est administrée sous forme d'une pulvérisation nasale pour le traitement des rhinites allergiques et des rhino-conjonctivites (J.F. Dessanges et al., Allergy et Clin. Immunol. News (1994), Suppl. n° 2, abrégé 1864; C. De Vos et al., Allergy and Clin Immunol. News (1994), Suppl. n° 2, abrégé 428). Une autre étude de pharmacologie clinique récente a montré que l'éflétirizine donnait des résultats étonnamment bons dans le traitement de l'urticaire, de la dermatite atopique et du prurit. Dans le brevet européen EP-B-1 034 171 au nom de la demanderesse, deux formes cristallines pseudopolymorphiques du dichlorhydrate d'éflétirizine, à savoir le dichlorhydrate d'éflétirizine anhydre et le dichlorhydrate d'éflétirizine monohydraté ont été découvertes et décrites pour leurs propriétés physiques particulièrement intéressantes.

Dans la demande de brevet international WO 98/41194 , une composition pharmaceutique administrable par voie orale est décrite, elle comprend au moins une couche comprenant une substance active et des excipients qui permettent la libération immédiate de ladite substance active après administration, et au moins une deuxième couche qui permet la libération contrôlée de la même ou d'une deuxième substance active, comprenant ladite même ou deuxième substance active, au moins un excipient de type matriciel, et au moins un agent alcalinisant. La composition comprend entre 5 et 50% en poids, par rapport au poids total de la composition d'agent alcalinisant soluble dans une phase aqueuse dans des conditions de pH physiologique. A cause de la présence de l'agent alcalinisant, cette composition a montré un bon profil de stabilité.

En raison d'un intérêt thérapeutique croissant pour l'éflétirizine, il a été entrepris de préparer de nouvelles compositions pharmaceutiques contenant ce principe actif.

Afin de faciliter le traitement du patient, il est souhaitable de développer de nouvelles compositions pharmaceutiques administrables par voie orale et de contrôler la libération des substances pharmaceutiquement actives de

manière à ce qu'elles puissent être administrées en une seule prise journalière tout en présentant une activité thérapeutique rapide.

Il est connu que la biodisponibilité de certains principes actifs peut être modifiée au moyen d'une formulation à libération prolongée qui libère le principe actif progressivement tout au long du tractus gastro-intestinal, sur une plus longue période et évite ainsi au malade l'absorption répétée d'une composition pharmaceutique.

Il a toutefois été établi que de telles formulations pharmaceutiques à libération prolongée couramment utilisées par l'homme du métier et appliquées à l'éflétirizine, ont le désavantage d'atteindre la concentration plasmatique efficace plus tardivement et donc de retarder l'action thérapeutique du principe actif. De plus, il a été constaté que de telles formulations à libération prolongée, comparativement à l'administration de deux doses à libération immédiate à 12 heures d'intervalle, induisaient une diminution de la concentration plasmatique maximale en éflétirizine ainsi qu'une diminution de sa biodisponibilité. En effet, les différentes études menées ont en outre révélé que l'éflétirizine présente une meilleure absorption dans les portions hautes du tractus gastrointestinal. Or pour soulager au plus vite le patient, il est souhaitable de lui délivrer rapidement une dose thérapeutiquement efficace d'éflétirizine tout en maintenant une concentration minimale efficace le plus longtemps possible et de préférence aux environs de 24 heures. De plus, toutes ces conditions doivent être réalisées, en maintenant une bioéquivalence avec 2 administrations de 5 à 25 mg d'éflétirizine sous une forme à libération immédiate à 12 heures d'intervalle.

Dans un souci de développer de nouvelles compositions pharmaceutiques administrables par voie orale qui puissent être administrées en une seule prise journalière, il a été découvert que l'association entre une fraction permettant une libération immédiate du principe actif et une seconde fraction permettant une libération prolongée du principe actif, permet de répondre idéalement aux exigences pharmacocinétiques particulières liées à l'utilisation de l'éflétirizine et à un meilleur contrôle de l'effet d'une prise de repas. Une grande difficulté a été de trouver le bon équilibre entre la libération immédiate du principe actif, et la libération prolongée permettant de maintenir une dose efficace durant une longue période tout en évitant d'atteindre le pic de concentration plasmatique engendrant des effets secondaires.

Les nouvelles compositions ainsi développées, ont en outre montré de manière particulièrement surprenante qu'en alliant au moins une fraction à libération immédiate et au moins une fraction à libération prolongée, les compositions pharmaceutiques ainsi obtenues permettaient de réduire de façon plus ou moins importante, suivant la répartition du principe actif entre les fractions, l'influence de la prise d'un repas avant leur absorption par le patient, cet effet n'étant absolument pas observé pour les compositions à libération immédiate. Cette découverte inattendue est particulièrement utile pour maintenir la bioéquivalence et la concentration plasmatique maximale d'une composition pharmaceutique, qu'elle soit prise avant ou après le repas, et de ce fait on réduit les conséquences d'une mauvaise manipulation ou utilisation par le patient.

Par ailleurs, de façon surprenante, il a été montré qu'une composition contenant moins de 5 % d'agent alcalinisant, ou d'en contenant pas, peut être préparée et que l'absorption se révèle constante et indépendante du pH.

Ainsi, la présente invention concerne de nouvelles compositions pharmaceutiques administrables par voie orale, contenant l'éflétirizine comme principe actif, caractérisées en ce qu'elles associent au moins une fraction permettant une libération immédiate du principe actif et au moins une fraction permettant une libération prolongée du principe actif, les quantités respectives de principe actif dans les deux fractions étant les valeurs comprises sur ou entre les deux droites définies par les équations suivantes;

$$Y = -0{,}6786X + 56{,}675$$

$$Y = -0{,}6636X + 7{,}975$$

dans lesquelles,

Y représente la quantité de principe actif en mg dans la fraction à libération immédiate, et
X représente la quantité de principe actif en mg dans la fraction à libération prolongée,
la quantité totale X + Y étant comprise entre 10 et 70 mg.

La quantité totale d'éflétirizine dans la composition est comprise entre 10 et 70 mg, et le rapport massique des quantités de principe actif entre la fraction à libération immédiate et la fraction à libération prolongée est compris entre 3 et 0,025. La quantité de principe actif est exprimée en quantité d'éflétirizine sous sa forme dichlorhydrate.

De telles compositions à libération modifiée peuvent êtres adaptées pour des administrations d'une à trois prises par jour.

Enfin, poursuivant la recherche d'une composition idéale pour limiter le nombre d'administrations journalières,

il a été mis en évidence des rapports quantitatifs particuliers de principe actif réparti entre la fraction à libération immédiate et la fraction à libération prolongée afin de répondre aux diverses exigences mentionnées ci-dessus en étant bioéquivalent à deux administrations de formes à libération immédiate tout en contrôlant l'influence d'une prise de repas. Ces rapports quantitatifs permettent d'obtenir des compositions pharmaceutiques particulièrement peu sensibles à la prise d'un repas.

De préférence, la présente invention se rapporte à une composition orale qui puisse être administrée en une seule prise journalière.

La teneur en éflétirizine dans la composition de l'invention est basée sur la quantité de dichlohydrate d'éflétirizine anhydre. Les teneurs peuvent varier selon le type d'éflétirizine mis en oeuvre (teneur en eau, type de sel, forme, ...).

On entend par "principe actif", l'acide 2-[2-(4-(bis(4-fluorophényl)méthyl]-1-pipérazinyl]éthoxy]acétique (éflétirizine) ou un de ses sels pharmaceutiquement acceptables.

On entend par "éflétirizine", l'acide 2-(2-[4-[bis(4-fluorophényl)méthyl]-1-pipérazinyl]éthoxy]acétique ou un de ses sels pharmaceutiquement acceptables.

On entend par "sels pharmaceutiquement acceptables", non seulement les sels d'addition d'acides non toxiques pharmaceutiquement acceptables tels que l'acide acétique, citrique, succinique, ascorbique, chlorhydrique, bromhydrique, sulfurique, phosphorique, etc., mais aussi les sels métalliques (par exemple sels de sodium ou de potassium) ou les sels d'ammonium. De préférence on utilise l'éflétirizine dichlorhydrate.

En outre, les différentes formes cristallines d'éflétirizine ou de ses sels pharmaceutiquement acceptables peuvent être utilisées selon la présente invention, par exemple, les formes anhydre et hydratée du dichlorhydrate d'éflétirizine, les formes cristallines pseudopolymorphiques du dichlorhydrate d'éflétirizine, à savoir le dichlorhydrate d'éflétirizine anhydre et le dichlorhydrate d'éflétirizine monohydraté.

On entend par "fraction permettant une libération immédiate ", une composition pharmaceutique qui va libérer le principe actif très rapidement après son absorption et en général dans les portions hautes du tractus gastro-intestinal.

On entend par "fraction permettant une libération prolongée", une composition pharmaceutique permettant de répartir la libération du principe actif sur une période plus ou moins longue par rapport à une fraction permettant une libération immédiate.

On entend par "composition à libération modifiée ", une composition associant deux types de libérations différents, dans notre cas une libération immédiate et une libération prolongée.

On entend par "biodisponibilité" la vitesse et la quantité de principe actif absorbée à partir de sa forme pharmaceutique pour devenir disponible au niveau de son site d'action pharmacologique.

Deux compositions pharmaceutiques sont dites "bioéquivalentes" si elles sont pharmaceutiquement équivalentes ou des alternatives pharmaceutiques et si leur biodisponibilité (Aires sous la courbe (AUC) non statistiquement différentes) respective après administration est suffisamment proche pour obtenir une efficacité thérapeutique équivalente.

Les compositions pharmaceutiques à libération immédiate ou à libération prolongée sont bien connues dans la littérature et devraient toutes permettre la mise en oeuvre de la présente invention. Il est important de souligner que les effets bénéfiques de l'invention devraient êtres observés, quels que soient les types de fractions à libération immédiate et à libération prolongée que le spécialiste du domaine technique décide d'associer.

Le contrôle de la libération de substances actives par la fraction à libération prolongée, lors de l'administration par voie orale peut se faire au moyen de compositions pharmaceutiques de type matriciel. Selon les excipients utilisés, on distingue trois types de matrices: les matrices inertes, hydrophiles et lipophiles. Par association d'excipients de ces différents types de matrices, on peut aussi créer des matrices mixtes. De nombreux autres moyens efficaces pour contrôler la libération de substances actives sont décrits dans la littérature tels que par exemple, les formes galéniques avec un enrobage barrière, les formes osmotiques, les formes flottantes ou les formes bioadhésives.

De préférence, dans la composition selon l'invention, la fraction permettant une libération prolongée de l'éflétirizine contient moins de 5 % en poids d'agent alcalinisant, poids par rapport au poids total de la fraction.

Les matrices inertes comprennent des excipients appartenant essentiellement à la classe des polymères thermoplastiques. Ils sont inertes vis-à-vis des tissus biologiques, des autres excipients dans la formulation et du principe actif. Ils sont insolubles et non-digestibles dans les fluides du tractus gastro-intestinal. Parmi ceux-ci, on peut citer le chlorure de polyvinyle, le polyéthylène, les copolymères d'acétate et de chlorure de vinyle, les polyméthylméthacrylates, les polyamides, les silicones, l'éthylcellulose, le polystyrène etc. Ils s'utilisent généralement à une concentration allant de 20 à 95%.

Les matrices hydrophiles comprennent des excipients gélifiants se répartissant en trois classes: les dérivés cellulosiques (hydroxypropylméthylcellulose, hydroxyéthylcellulose, hydroxypropylcellulose, méthylcellulose etc.), les polysaccharides non cellulosiques (galactomannes, gomme guar, gomme caroube, gomme arabique, gomme sterculia, agar agar, alginates etc.) et les polymères de l'acide acrylique (carbopols 934P et 974P etc.). Ils s'utilisent généralement à une concentration de 20 à 70%.

Les matrices lipidiques comprennent des excipients gras de quatre types: les glycérides (mono- di- ou triglycérides: stéarine, palmitine, laurine, myristine, huiles de ricin ou de coton hydrogénées, précirol etc.), les acides et les alcools gras (acides stéarique, palmitique, laurique; alcools stéarylique, cétylique, cétostéarylique etc.), les esters d'acides gras (monostéarates de propylène glycol et de saccharose, distéarate de saccharose etc.) et les cires (cire blanche, cire de cachalot etc.). Ils s'utilisent généralement à une concentration de 10 à 50%.

En ce qui concerne, les excipients permettant la libération immédiate du principe actif, ils peuvent être choisis parmi les diluants (phosphate calcique, lactose etc.), les liants (cellulose microcristalline, amidons, polyvinylpyrrolidone etc.), les désintégrants (amidons et amidons modifiés, dérivés cellulosiques, dérivés alginiques, pectines etc.), les lubrifiants et agents d'écoulement (talc, stéarate de magnésium, silice colloïdale etc.), les agents de masquage de goût ($\alpha$-cyclodextrine, $\beta$-cyclodextrine, $\gamma$-cyclodextrine et leurs dérivés), des arômes ou des colorants.

Outre les composants précités, les compositions pharmaceutiques selon la présente invention peuvent également contenir d'autres excipients tels que des diluants (phosphate calcique, lactose etc.), des liants (cellulose microcristalline, amidons, polyvinylpyrrolidone etc.), des désintégrants (amidons et amidons modifiés, dérivés cellulosiques, dérivés alginiques, pectines etc.), des lubrifiants et agents d'écoulement (talc, stéarate de magnésium, silice colloïdale etc.), des agents de masquage de goût ($\alpha$-cyclodextrine, $\beta$-cyclodextrine, $\gamma$-cyclodextrine et leurs dérivés), des arômes ou des colorants ainsi que des agents de pelliculage (exemple: dérivés cellulosiques, résines méthacryliques, chlorure de polyvinyle, nylons, etc.).

Les compositions pharmaceutiques selon la présente invention peuvent se présenter sous une forme solide et liquide. Il est important de souligner que les effets bénéfiques de l'invention devraient être observés, quelle que soit la présentation de la forme galénique. Les compositions pharmaceutiques selon la présente peuvent se présenter sous la forme de sirops, comprimés, comprimés multicouches, granules, microgranules, capsules, gélules etc., ces formes étant enrobées ou non.

Les formes pharmaceutiques de type gélules ou capsules peuvent par exemple contenir un mélange de granules à libération immédiate et prolongée. Des granules à libération prolongée peuvent également être mélangés à une formulation à libération immédiate et comprimés en un comprimé.

La forme pharmaceutique peut se présenter, par exemple, comme une gélule, transparente ou opaque, contenant deux comprimés dont l'un renferme la fraction permettant une libération immédiate et l'autre renferme fraction permettant une libération prolongée (comprimé pouvant être recouvert d'une pellicule qui permette la libération prolongée).

Une autre présentation peut envisager des gélules contenant un mélange de microgranules dont certains pouvant être recouverts par un pelliculage permettent la libération prolongée du principe actif et les autres permettent une libération immédiate du principe actif.

La forme pharmaceutique contenant l'association selon la présente invention se présente de préférence comme un comprimé double ou multicouches, plus particulièrement comme comprimés préparés par soumission à plus d'une compression. Le résultat d'une telle forme peut être soit celui d'un comprimé à deux ou plusieurs couches, soit celui d'un comprimé à l'intérieur d'un autre comprimé, les deux parties libérant le principe actif de façon différente.

De préférence, lorsque ces deux types de fractions sont présentes dans une forme de comprimé bicouche, les compositions pharmaceutiques administrables par voie orale comprennent ;

A. au moins une couche comprenant le principe actif et des excipients qui permettent la libération immédiate du dit principe actif après administration, et

B. au moins une deuxième couche qui permet la libération contrôlée du même principe actif, comprenant le principe actif et au moins un excipient qui permette de retarder sa libération.

De telles compositions pharmaceutiques combinées peuvent être préparées selon diverses méthodes connues de l'homme du métier.

Plus particulièrement, ces compositions pharmaceutiques combinées peuvent se présenter sous la forme d'un comprimé dans lequel au moins une couche A est accolée à au moins une couche B. Dans ce cas, de telles compositions pharmaceutiques peuvent être préparées par un procédé comprenant les étapes successives suivantes:

1) préparation de mélanges homogènes séparés à partir des composants des couches A et B, et
2) compression des mélanges homogènes obtenus en 1) dans une comprimeuse multicouches.

Eventuellement, la préparation des mélanges homogènes obtenus à l'étape 1) peut contenir une étape de granulation de certains composants.

Les comprimeuses multicouches permettant de préparer ce genre de comprimés sont les comprimeuses multicouches de type Courtoy, Manesty, Hata, Fette, Killian, etc.

Les comprimés multicouches sont particulièrement bien adaptés à la mise en oeuvre de la présente invention.

Les recherches menées lors du développement de nouvelles compositions pharmaceutiques administrables

en une seule prise journalière, ont mis en évidence une répartition particulière du principe actif entre la fraction à libération immédiate et la fraction à libération prolongée. En effet, la présente invention concerne tout particulièrement des compositions comprenant de 10 à 70 mg d'éflétirizine, dans lesquelles le rapport des quantités du principe actif entre la fraction à libération immédiate et la fraction à libération contrôlée est compris entre 3 et 0,025 et de préférence entre 1,6 et 0,05. La détermination des deux équations, définies ci-dessus, délimitant les quantités idéales d'éflétirizine dans chacune des fractions de la composition telle que définie par l'invention a été élaborée à partir des données présentées dans l'exemple 6.

Un autre avantage inattendu et surprenant selon la présente invention, est que l'association d'au moins une fraction à libération immédiate et d'au moins une fraction à libération contrôlée permet de limiter ou empêcher la chute de la concentration plasmatique maximale ($C_{max}$) ainsi que de limiter ou empêcher la modification de la biodisponibilité de l'éflétirizine, que le patient prenne ou non un repas avant l'ingestion du médicament, l'efficacité de ce système étant évidemment liée à la répartition du principe actif entre les fractions. Ainsi, la présente invention concerne tout particulièrement des compositions comprenant de 10 à 70 mg d'éflétirizine, dans lesquelles le rapport des quantités de principe actif entre la fraction à libération immédiate et la fraction à libération contrôlée est compris entre 3 et 0,025. Le maintien de la concentration plasmatique maximale ainsi que celui de la biodisponibilité de l'éflétirizine est particulièrement observé pour les nouvelles compositions pharmaceutiques répondants aux équations présentées ci-dessus.

Les principaux paramètres pharmacocinétiques révélant que la prise d'un repas ne fait pas chuter le $C_{max}$ et que la biodisponibilité n'est pas significativement modifiée pour les compositions selon l'invention sont exposés dans les exemples 4, 5 et 6. L'exemple 4 montre quant à lui une influence très marquée d'une prise de repas sur les paramètres pharmacocinétiques d'une composition à libération immédiate: le $C_{max}$ est diminué de 42 %. La biodisponibilité est également diminuée.

Les exemples qui suivent illustrent la présente invention. De telles compositions pharmaceutiques combinant une fraction à libération immédiate avec une fraction à libération prolongée peuvent être préparées selon diverses méthodes conventionnelles bien connues dans la littérature spécialisée.

Les abréviations suivantes sont utilisées. LP : libération prolongée ; LI : libération immédiate; $C_{max}$: concentration plasmatique maximale; $t_{max}$: le temps mis pour atteindre le $C_{max}$ ; AUC : aire sous la courbe ; L'AUC caractérise la biodisponibilité et peut être exprimé en nanogramme heure par millilitre ou en % par rapport à la biodisponibilité de la solution de référence.

Exemple 1. Comprimés à libération prolongé (LP) classiques dosés à 30 mg d'éflétirizine

Neuf formulations de comprimés dosés à 30 mg d'éflétirizine ont été réalisées et leur composition est reprise dans le tableau 1. L'éflétirizine est granulée avec un ou plusieurs excipients et à ce granulé sont ajoutés les autres excipients (phase externe). Le mélange est ensuite comprimé.

Tableau 1. Composition des comprimés LP classiques (A à I).

| Composantsmg/ comprimé | | | | | | | |
|---|---|---|---|---|---|---|---|
| | A | B | C | F | G | H | I |
| Granulé : | | | | | | | |
| Eflétirizine dichlorhydrate | 30 | 30 | 30 | 30 | 30 | 30 | 30 |
| Phosphate calcique dibasique hydraté (Emcompress™) | 70 | 145,5 | 97 | 35 | - | - | 59 |
| Hydroxypropylméthylcellulose[1] (Methocel K15 MCR™)[2] | - | 45 | 25 | - | - | - | - |
| Beta-cyclodextrine | - | - | - | - | 82 | 82 | - |
| Phase externe : | | | | | | | |
| Hydroxypropylméthylcellulose[1] (Methocel K100 MCR™)[3] | 160 | - | - | - | - | - | - |
| Hydroxypropylméthylcellulose[1] (Methocel K15 MCR™)[2] | - | 75 | 75 | 33,3 | 70 | - | - |
| Hydroxypropylméthyl Cellulose[1] (Methocel E4 MCR™)[4] | - | - | - | - | - | 40 | 50 |
| - Cellulose microcristalline (Avicel pH 102™) | - | - | - | - | - | 45 | - |
| Phosphate calcique dibasique hydraté (Emcompress™) | 134 | - | - | - | 15 | - | 13 |
| Bicarbonate de sodium | - | - | - | - | - | - | - |
| Stéarate de magnésium | 4 | 3 | 2 | 1 | 2 | 2 | 2 |

(suite)

| | | | | | | | |
|---|---|---|---|---|---|---|---|
| Phase externe :<br>Silice colloïdale (Aérosil 200™) | 2 | 1,5 | 1 | 0,7 | 1 | 1 | 1 |

[1] USP substitution type : 2208
[2] viscosité nominale (méthode EP) = 7382 mPa.s
[3] viscosité nominale (méthode EP) = 18243 mPa.s
[4] viscosité nominale (méthode EP) = 3000 mPa.s (USP : HPMC 2910)

Ces comprimés ont fait l'objet de 3 études de biodisponibilité sur 8 volontaires reprenant les comprimés A, B et C (étude A225), D (étude A231) et E, F, et G (étude A244). La référence utilisée est chaque fois une solution d'éflétirizine administrée au même dosage. Les principaux paramètres pharmacocinétiques relatifs à ces études sont repris dans les tableaux 2 à 4.

Tableau 2. Principaux paramètres pharmacocinétiques de l'étude de biodisponibilité A225.

| Paramètres | Solution | A | B | C |
|---|---|---|---|---|
| $C_{max}$ (ng/ml) | 526 | 111 | 139 | 150 |
| $t_{max}$ (h) | 1 | 4 | 3,87 | 3,5 |
| AUC (ng.h/ml) | 2777 | 1603 | 1880 | 2159 |
| AUC (% solution) | 100 | 57,7 | 67,7 | 77,4 |

Tableau 3. Principaux paramètres pharmacocinétiques de l'étude de biodisponibilité A231.

| Paramètres | Solution | D |
|---|---|---|
| $C_{max}$(ng/ml) | 561 | 158 |
| $t_{max}$ (h) | 0,88 | 3,52 |
| AUC(ng.h/ml) | 2891 | 2242 |
| AUC (% solution) | 100 | 77,6 |

Tableau 4. Principaux paramètres pharmacocinétiques de l'étude de biodisponibilité A244.

| Paramètres Solution EFG | | | | |
|---|---|---|---|---|
| $C_{max}$ (ng/ml) | 583 | 146 | 204 | 165 |
| $t_{max}$ (h) | 0,75 | 4 | 4 | 3,5 |
| AUC (ng.h/ml) | 2827 | 1717 | 1989 | 1775 |
| AUC (% solution) | 100 | 60,7 | 70,4 | 63 |

Pour toutes les formes LP, on peut noter un $C_{max}$ environ 3 à 5 fois plus faible que celui correspondant à la solution de référence. L'AUC est aussi plus faible : pour les comprimés LP, elle n'atteint que 60 à 80 % de celle de la solution de référence.

Exemple 2. Administration de 2 fois 15 mg d'éflétirizine à libération immédiate (LI) à 12 heures d'intervalle.

Deux fois 15 mg d'éflétirizine en solution ont été administrés à 12 volontaires sains à 12 heures d'intervalle. Les principaux paramètres pharmacocinétiques sont repris dans le tableau 5.

Tableau 5. Principaux paramètres pharmacocinétiques après administration de 2 fois 15 mg d'éflétirizine en solution à 12 heures d'intervalle.

| Paramètre | |
|---|---|
| $C_{max}$ (1) (ng/ml) | 307 |
| $C_{max}$ (2) (ng/ml) | 305 |
| $t_{max}$ (1) (h) | 0,6 |

(suite)

| Paramètre | |
|---|---|
| $t_{max}$ (2) (h) | 12,6 |
| AUC (ng.h/ml) | 2800 |

Exemple 3. Simulations de pharmacocinétiques à partir des résultats des exemples 1 et 2.

Compte tenu des cinétiques observées pour les formes LP (exemple 1)et LI (exemple 2) et compte tenu du fait que l'on a pu montrer qu'il était nécessaire d'associer une forme LI à une forme LP pour obtenir un $C_{max}$ suffisant, il a été procédé à des simulations. Ces simulations ont porté sur des doses à administrer sous forme LI (5 et 10 mg) et sous forme LP (20, 25 et 30 mg) d'éflétirizine afin d'obtenir un $C_{max}$ et une AUC proche de ceux observés après administration de 2 fois 15 mg à 12 heures d'intervalle. Les formulations LP choisies pour ces simulations ont été celles qui donnaient les meilleures AUC dans l'exemple 1, soit les formulations C, D et F. Les principaux paramètres cinétiques issus de ces simulations sont repris dans les tableaux 6 à 10.

Tableau 6. Principaux paramètres pharmacocinétiques après simulations en se basant sur les cinétiques observées dans les exemples 1 et 2. Doses LI/LP : 5/25 mg.

| | Formulations LP | | |
|---|---|---|---|
| Paramètres | C | D | F |
| $C_{max}$ (ng /ml) | 186,2 | 187,5 | 219,4 |
| $t_{max}$ (h) | 3,0 | 2,0 | 3,0 |
| AUC (ng.h/ml) | 2201 | 2369 | 2083 |

Tableau 7.Principaux paramètres pharmacocinétiques après simulations en se basant sur les cinétiques observées dans les exemples 1 et 2. Doses LI/LP : 10/20 mg.

| Formulations LP | | | |
|---|---|---|---|
| Paramètres | C | D | F |
| $C_{max}$ (ng/ml) | 233,9 | 246,5 | 258,4 |
| $t_{max}$ (h) | 1,5 | 1,5 | 2,0 |
| AUC (ng.h/ml) | 2297 | 2404 | 2266 |

Tableau 8. Principaux paramètres pharmacocinétiques après simulations en se basant sur les cinétiques observées dans les exemples 1 et 2. Doses LI/LP : 10/25 mg.

| Formulations LP | | | |
|---|---|---|---|
| Paramètres | C | D | F |
| $C_{max}$ (ng/ml) | 250,8 | 266,6 | 286,7 |
| $t_{max}$ (h) | 1,5 | 1,5 | 2,0 |
| AUC (ng.h/ml) | 2645 | 2788 | 2548 |

Tableau 9. Principaux paramètres pharmacocinétiques après simulations en se basant sur les cinétiques observées dans les exemples 1 et 2. Doses LI/LP : 5/30 mg.

| Formulations LP | | | |
|---|---|---|---|
| Paramètres | C | D | F |
| $C_{max}$ (ng/ml) | 211,9 | 210,4 | 251,7 |
| $t_{max}$ (h) | 3,0 | 2,0 | 3,0 |
| AUC (ng.h/ml) | 2553 | 2762 | 2407 |

Tableau 10. Principaux paramètres pharmacocinétiques après simulations en se basant sur les cinétiques observées dans les exemples 1 et 2. Doses LI/LP : 10/30 mg.

| Formulations LP | | | |
|---|---|---|---|
| Paramètres | C | D | F |
| $C_{max}$ (ng/ml) | 269,7 | 286,6 | 314,9 |
| $t_{max}$ (h) | 3,0 | 1,5 | 2,0 |
| AUC (ng.h/ml) | 2995 | 3175 | 2871 |

Les meilleurs résultats, tant pour le $C_{max}$ que pour l'AUC, sont obtenus pour le dosage LI/LP de 10/25 mg, notamment à partir de la formulation LP : D (tableau 8).

Exemple 4. Administration de 60 mg d'éflétirizine LI : influence d'un repas.

Une gélule contenant 60 mg d'efleterizine a été administrée à 20 patients à jeun et à 22 patients qui ont préalablement pris un repas gras standardisé. Les principaux paramètres pharmacocinétiques sont repris dans le tableau 11.

Tableau 11. Principaux paramètres pharmacocinétiques après administration de 60 mg d'éflétirizine à jeun ou après repas.

| Paramètres | A jeun | Après repas |
|---|---|---|
| $C_{max}$ (ng/ml) | 1474 | 855 |
| $t_{max}$ (h) | 0,77 | 3,03 |
| AUC (ng.h/ml) | 6354 | 5784 |

On peut noter une influence très marquée d'une prise de repas : le $C_{max}$ est diminué de 42 %. La biodisponibilité est également diminuée.

Exemple 5. Administration de 10 mg d'éflétirizine LI conjointement à un comprimé LP dosé à 25 mg.

Au cours d'un essai croisé, 12 volontaires ont reçu 3 types de traitements :

- 10 mg d'éflétirizine LI sous forme de solution et un comprimé LP de 25 mg d'élétirizine (J) à jeun
- 10 mg d'éflétirizine LI sous forme de solution et un comprimé LP de 25 mg d'éflétirizine (J) après un repas gras standardisé
- 15 mg d'éflétirizine LI sous forme de solution 2 fois à 12 heures d'intervalle.

La formule du comprimé LP à 25 mg de principe actif (J) utilisé dans cette étude est reprise dans le tableau 12. Les 3 premiers composants sont granulés. Les 3 derniers sont ensuite ajoutés et le mélange est comprimé. Le comprimé J reprend la composition du comprimé F à laquelle il a été ajouté un agent de granulation, la Povidone K 30™.

Tableau 12. Composition du comprimé LP dosé à 25 mg d'éflétirizine (J).

| Composants | Comprimé J mg/ comprimé |
|---|---|
| Eflétirizine dichlorhydrate | 25 |
| Phosphate calcique dibasique hydraté (Emcompress™) | 30 |
| Polyvinylpyrrolidone (Povidone K 30™) | 1,7 |
| Hydroxypropylméthylcellulose (Methocel K 15MCR™) | 28,4 |
| Silice colloïdale (Aerosil 200™) | 0,6 |
| Stéarate de magnésium | 0,8 |

Les principaux paramètres cinétiques sont repris dans le tableau 13.

Tableau 13. Principaux paramètres pharmacocinétiques après administration de 10 mg d'éflétirizine LI et de 25 mg d'éflétirizine LP à jeun et après repas en comparaison avec l'administration de 2 fois 15 mg d'éflétirizine LI à 12 heures d'intervalle.

| | LI/LP 10/25 mg | | LI 15 mg x 2 |
|---|---|---|---|
| Paramètre | à jeun | après repas | |
| $C_{max}$ (1) (ng/ml) | 280 | 355 | 307 |
| $C_{max}$ (2) (ng/ml) | | | 305 |
| $t_{max}$ (1) (h) | 1,5 | 4 | 0,6 |
| $t_{max}$ (2) (h) | | | 12,6 |
| AUC (ng.h/ml) | 2680 | 3031 | 2801 |

D'une manière surprenante, contrairement à ce qui avait été observé dans l'exemple 4, la prise d'un repas ne fait pas chuter le $C_{max}$ et la biodisponibilité n'est pas significativement modifiée.

D'une manière surprenante aussi, malgré l'absence d'agent alcalinisant dans la formulation LP, l'absorption in vivo se révèle constante et donc indépendante du pH contrairement à ce qui avait été observé in vitro dans les tests de dissolution décrits dans la demande de brevet PCT/BE98/00033. Cette indépendance du pH se traduit par de faibles variations inter-sujets après administration de la forme LI/LP et les coefficients de variation sur l'AUC sont respectivement de 23 % et 17 % à jeun et après repas.

Exemple 6. Simulation des surfaces sous la courbe pour des associations de formes LI et LP d'éflétirizine.

A partir des pharmacocinétiques obtenues dans l'exemple 5, des simulations ont été réalisées pour des associations à différentes concentrations en éflétirizine de formes LI et LP. Pour chacune de ces associations (forme LI de 0 à 20 mg et forme LP de 15 à 35 mg) la surface sous la courbe a été calculée en pourcentage par rapport à celle obtenue pour la référence LI 2 fois 15 mg. La limite de confiance au seuil de 90 % pour ces pourcentages a été calculée et est de 7 %.

Peuvent être considérées comme bioéquivalentes, les compositions dont la surface sous la courbe exprimée en pourcentage, compte tenu d'une variabilité de 7%, se situent dans les limites de 80 à 125 %.

A partir des données obtenues, une "modélisation" reprenant les différentes compositions possibles en libération immédiate et retardée, bioéquivalentes à 2 x 15mg d'une forme à libération immédiate a permis de calculer les équations de deux droites de régression à partir des compositions directement inférieures et supérieures aux limites de confiance (80-125%).

Les deux droites obtenues sont définies par les équations suivantes :

$$Y = -0,6786X + 34 \qquad (R^2 = 0.9918)$$

$$Y = -0,6636X + 23,924 (R^2 = 0.9955)$$

pour lesquelles,

Y représente la quantité de principe actif en milligrammes (mg) dans la fraction à libération immédiate, et
X représente la quantité de principe actif en milligrammes (mg) dans la fraction à libération prolongée ,
$R^2$ représente les coefficients de corrélation pour les deux droites.

Les valeurs en-dessous de la droite d'équation Y = -0,6636X + 34 correspondent à des composition de biodisponibilité trop faibles et celles au-dessus de la droite d'équation Y = -0,6786X + 23,924 correspondent à des compositions de biodisponibilité trop élevées. Sont donc à prendre en considération les valeurs comprises sur ou entre les deux droites, qui sont donc bioéquivalentes à l'administration d'une forme à libération immédiate de 2 fois 15 mg LI.

Les pharmacocinétiques étant linéaires, les équations des droites délimitant les compositions bioéquivalentes à deux administrations à 12 heures d'intervalle d'autres doses, soit Z mg, deviennent :

$$Y = -0,6786X + 34\ Z/15 \quad \text{ou} \quad Y = -0,6786X + 2,267Z \quad \text{(Eq. 1)}$$

$$Y = -0,6636X + 23,924\ Z/15 \quad \quad Y = -0,6636X + 1,595Z \quad \text{(Eq. 2)}$$

Z étant compris entre 5 et 25 et de préférence entre 7 et 15.

Ainsi, pour Z = 25, Eq. 1 devient :

$$Y = -0,6786X + 56,675$$

et pour Z = 5, Eq. 2 devient :

$$Y = -0,6636X + 7,975$$

Exemple 7. Préparation bioéquivalente à 2 administrations de 10 mg d'éflétirizine sous forme à libération immédiate à 12 h d'intervalle

Tableau 14. Composition d'un comprimé dosé à 23,4 mg d'éflétirizine bioéquivalent à 2 administrations de 10 mg d'éflétirizine sous forme à libération immédiate à 12 h d'intervalle.

| Composants | mg/ comprimé |
|---|---|
| Couche LP : | |
| Eflétirizine dichlorhydrate | 16,7 |
| Phosphate calcique dibasique hydraté (Emcompress™) | 30,0 |
| Hydroxypropylméthylcellulose (Methocel K15 MCR™) | 28,6 |
| Cellulose microcristalline (Avicel PH 101™) | 8,3 |
| Silice colloïdale (Aerosil 200™) | 0,6 |
| Magnésium stéarate 0,8 | |
| Couche LI : | |
| Eflétirizine dichlorhydrate | 6,7 |
| Lactose monohydrate | 45,6 |
| Cellulose microcristalline (Avicel PH 102™) | 26,5 |
| Silice colloïdale (Aerosil 200™) | 0,4 |
| Magnésium stéarate | 0,8 |
| Enrobage : | |
| Hydroxypropylméthylcellulose + dioxyde de titane (White Opadry Y-1-7000™) | 4,95 |

Exemple 8. Préparation bioéquivalente à 2 administrations de 7 mg d'éflétirizine sous forme à libération immédiate à 12 h d'intervalle

Tableau 15. Composition d'un comprimé dosé à 16,4 mg d'éflétirizine bioéquivalent à 2 administrations de 7 mg d'éflétirizine sous forme à libération immédiate à 12 h d'intervalle.

| Composants | mg/ comprimé |
|---|---|
| Couche LP : | |
| Eflétirizine dichlorhydrate | 11,7 |

(suite)

| Composants | mg/ comprimé |
|---|---|
| Couche LP : | |
| Phosphate calcique dibasique hydraté (Emcompress™) | 30,0 |
| Hydroxypropylméthylcellulose (Methocel K15 MCR™) | 28,6 |
| Cellulose microcristalline (Avicel PH 101™) | 13,3 |
| Silice colloïdale (Aerosil 200™) | 0,6 |
| Magnésium stéarate | 0,8 |
| Couche LI : | |
| Eflétirizine dichlorhydrate | 4,7 |
| Lactose monohydrate | 46,6 |
| Cellulose microcristalline (Avicel PH 102™) | 27,5 |
| Silice colloïdale (Aerosil 200™) | 0,4 |
| Magnésium stéarate | 0,8 |
| Enrobage : | |
| Hydroxypropylméthylcellulose + dioxyde de titane (White Opadry Y-1-7000™) | 4,95 |

## Revendications

1. Composition pharmaceutique, administrable par voie orale, contenant l'éflétirizine comme principe actif, **caractérisée en ce qu'**elle associe au moins une fraction permettant une libération immédiate de l'éflétirizine et au moins une fraction permettant une libération prolongée de l'éflétirizine, les quantités respectives de principe actif dans les deux fractions étant les valeurs comprises sur ou entre les deux droites définies par les équations suivantes

$$Y = -0{,}6786X + 56{,}675$$

$$Y = -0{,}6636X + 7{,}975$$

dans lesquelles

Y représente la quantité de l'éflétirizine en milligrammes (mg) dans la fraction à libération immédiate, et
X représente la quantité de l'éflétirizine en milligrammes (mg) dans la fraction à libération prolongée : et

la quantité totale X + Y étant comprise entre 10 et 70 mg, et le rapport massique des quantités de principe actif entre la fraction à libération immédiate et la fraction à libération prolongée est compris entre 3 et 0,025.

2. Composition selon la revendication 1, **caractérisée en ce qu'**elle puisse être administrée en une seule prise journalière, en obtenant l'effet thérapeutique souhaité.

3. Composition selon la revendication 1 ou 2 **caractérisée en ce que** les deux fractions sont présentes sous forme de comprimé bicouche.

4. Composition selon l'une quelconque des revendications 1 à 3 **caractérisée en ce que** la fraction permettant une libération prolongée de l'éflétirizine contient moins de 5 % en poids d'agent alcalinisant, poids par rapport au poids total de la fraction.

## Claims

1. A pharmaceutical composition which can be administered orally, containing efletirizine as active principle, **characterized in that** it combines at least one fraction which allows immediate release of the efletirizine and at least one

fraction which allows prolonged release of the efletirizine, the respective amounts of active principle in the two fractions being the values included on or between the two straight lines defined by the following equations:

$$Y = -0.6786X + 56.675$$

$$Y = -0.6636X + 7.975$$

in which,

Y represents the amount of efletirizine in milligrams (mg) in the immediate-release fraction, and
X represents the amount of efletirizine in milligrams (mg) in the prolonged-release fraction, and
the total amount X + Y being between 10 and 70 mg; and the weight ratio of the amount of active principle in the immediate-release fraction to the amount of active principle in the prolonged-release fraction is between 3 and 0.025.

**2.** The composition as claimed in claim 1, **characterized in that** it can be administered in a single daily dose, while obtaining the desired therapeutic effect.

**3.** The composition as claimed in claim 1 or 2, **characterized in that** the two fractions are provided in the form of a two-layer tablet.

**4.** The composition as claimed in any one of claims 1 to 3, **characterized in that** the fraction which allows prolonged release of the efletirizine contains less than 5% by weight of basifying agent, weight relative to the total weight of the fraction.

## Patentansprüche

**1.** Pharmazeutische Zusammensetzung, die auf oralem Weg verabreichbar ist, die Efletirizin als Wirkstoff enthält, **dadurch gekennzeichnet, dass** sie mindestens eine Fraktion, die eine unmittelbare Freisetzung von Efletirizin gestattet, und mindestens eine Fraktion, die eine verlängerte Freisetzung von Efletirizin gestattet, vereinigt, wobei die jeweiligen Mengen des Wirkstoffs in den beiden Fraktionen Werte sind, die auf oder zwischen den beiden Geraden liegen, die durch die folgenden Gleichungen definiert sind:

$$Y = -0{,}6786X + 56{,}675$$

$$Y = -0{,}6636X + 7{,}975,$$

worin

Y die Menge an Efletirizin in Milligramm (mg) in der Fraktion mit unmittelbarer Freisetzung darstellt und
X die Menge an Efletirizin in Milligramm (mg) in der Fraktion mit verlängerter Freisetzung darstellt, und
die Gesamtmenge X + Y zwischen 10 und 70 mg liegt und das Gewichtsverhältnis der Mengen des Wirkstoffs zwischen der Fraktion mit unmittelbarer Freisetzung und der Fraktion mit verlängerter Freisetzung zwischen 3 und 0,025 beträgt.

**2.** Zusammensetzung nach Anspruch 1, **dadurch gekennzeichnet, dass** sie in einer einzigen Tagesdosis verabreicht werden kann, wobei die gewünschte therapeutische Wirkung erhalten wird.

**3.** Zusammensetzung nach Anspruch 1 oder 2, **dadurch gekennzeichnet, dass** die zwei Fraktionen in Form einer Zweischichttablette vorliegen.

4. Zusammensetzung nach einem der Ansprüche 1 bis 3, **dadurch gekennzeichnet, dass** die Fraktion, die eine verlängerte Freisetzung von Efletirizin gestattet, weniger als 5 Gew.-%, bezogen auf das Gesamt-gewicht der Fraktion, an Alkalisierungsmittel enthält.